# EUROPEAN PATENT SPECIFICATION

(11) **EP 4 255 427 B1**
(45) Date of publication and mention of the grant of the patent: **06.11.2024**
(21) Application number: 21820615.9
(22) Date of filing: 03.12.2021
(51) Int. Cl.: A61K 31/4365, A61K 45/06, A61P 13/12

(54) **CLOPIDOGREL FOR USE IN THE TREATMENT OF FOCAL SEGMENTAL GLOMERULOSCLEROSIS (FSGS)**
CLOPIDOGREL ZUR ANWENDUNG BEI DER THERAPIE VON FOKAL SEGMENTALE GLOMERULOSKLEROSE (FSGS)
CLOPIDOGREL POUR UTILISATION DANS LE TRAITEMENT DE LA HYALINOSE SEGMENTAIRE ET FOCAL

(30) Priority: 03.12.2020 EP 20211546
(43) Date of publication of application: 11.10.2023
(73) Proprietor: Delta 4 GmbH, 1080 Vienna (AT)
(72) Inventor: PERCO, Paul, 1080 Vienna (AT); GEBESHUBER, Christoph, 1080 Vienna (AT)
(74) Representative: Loidl, Manuela Bettina
(86) International application number: PCT/EP2021/084250
(87) International publication number: WO 2022/117862

(56) References cited:
- KR-A- 20180 121 722
- TU XIAOWEN ET AL: "Anti-inflammatory renoprotective effect of clopidogrel and irbesartan in chronic renal injury", JOURNAL OF THE AMERICAN SOCIETY OF NEPHROLOGY, WILLIAMS AND WILKINS, BALTIMORE, MD, US, vol. 19, no. 1, 1 January 2008 (2008-01-01), pages 77 - 83, XP002563147, ISSN: 1046-6673, [retrieved on 20071128], DOI: 10.1681/ASN.2007020160
- PETERS HARM ET AL: "Platelet inhibition limits TGF-[beta] overexpression and matrix expansion after induction of anti-thy1 glomerulonephritis", KIDNEY INTERNATIONAL, vol. 65, no. 6, 1 June 2004 (2004-06-01), GB, pages 2238 - 2248, XP055797655, ISSN: 0085-2538, DOI: 10.1111/j.1523-1755.2004.00630.x
- SU XIAOLE ET AL: "Effect of antiplatelet therapy on cardiovascular and kidney outcomes in patients with chronic kidney disease: a systematic review and meta-analysis", vol. 20, no. 1, 7 August 2019 (2019-08-07), XP055798111, Retrieved from the Internet <URL:http://link.springer.com/article/10.1186/s12882-019-1499-3/fulltext.html> DOI: 10.1186/s12882-019-1499-3
- AVI Z. ROSENBERG ET AL: "Focal Segmental Glomerulosclerosis", CLINICAL JOURNAL OF THE AMERICAN SOCIETY OF NEPHROLOGY, vol. 12, no. 3, 27 February 2017 (2017-02-27), pages 502 - 517, XP055513283, ISSN: 1555-9041, DOI: 10.2215/CJN.05960616

## Description

### FIELD OF THE INVENTION

The present invention relates to a pharmaceutical preparation comprising clopidogrel, or a salt or solvate thereof, for use in the treatment of focal segmental glomerulosclerosis (FSGS).

### BACKGROUND OF THE INVENTION

Focal segmental glomerulosclerosis (FSGS) is a severe glomerulopathy frequently leading to end stage kidney disease. FSGS is used to describe a histopathological lesion defined by the presence of sclerosis in some parts (segmental) of some glomeruli (focal). This lesion refers to both a disease characterized by primary podocyte injury, or secondary to other impacts including hypertension, obesity or viruses. FSGS can be found without an identifiable cause (idiopathic).

The most common manifestation of FSGS is proteinuria ranging from subnephrotic to nephrotic levels (heavy proteinuria, hypoalbuminemia and hyperlipidemia). Heavy proteinuria is associated with progressive loss of kidney function and kidney failure. It accounts for about 15% of end-stage renal disease (ESRD). Massive proteinuria (>10-15 g/day) leads to rapid deterioration of renal function and progression to ESRD within 2-3 years.

There is no approved drug for FSGS. The current standard of care for patients with FSGS include steroids, ACE inhibitors orARBs, immunosuppressive drugs such as glucocorticoids or by calcineurin inhibitors, if needed, for intolerance or inadequate response to glucocorticoids; diuretics, plasmapheresis, diet change and statins. WO2006029349A1 describes the use of PPAR agonists alone or in combination with inhibitors of platelet aggregation for the delay of progression or treatment of a diabetic disease or disorder, a hyperlipidemic disease or disorder, a metabolic disease or disorder and/or a cardiovascular disease or disorder or an addictive disease.

Tu X. et al., 2008, report the use of Clopidogrel in early renal injury caused by surgical kidney ablation of 5/6 of the kidney volume due to inhibition of renal inflammation.

Peters H. et al. (2004) refer to the analysis of the effect of clopidogrel on early injury and subsequent repair phase of experimental acute anti-thy1 glomerulonephritis in a rat model of acute glomerular wound repair.

Su X. et al (2019) review the effect of antiplatelet therapy on cardiovascular and kidney outcomes in patients with chronic kidney disease and describe that the application of antiplatelet agents might provide an overall net benefit.

KR20180121722A discloses a composition for preventing or treating a renal disease, which comprises pravastatin and an anti-platelet agent such as clopidogrel.

There are, however, only poor response rates. Renin-angiotensin-aldosterone (RAAS) blockers are also used to control proteinuria, an important signature of FSGS. Existing treatments, however, achieved only limited success.

About 5400 patients are diagnosed with FSGS every year in the United States, but the number of cases is rising more than any other cause of Nephrotic Syndrome. Approximately 1,000 FSGS patients receive kidney transplants every year. Within hours to weeks after a kidney transplant, however, FSGS returns in approximately 30-40% of patients. Only 20% of patients, however, achieve complete remission after 5 years of treatment, and 40% of patients show no remission (Troyanov S. et al., 2005).

Despite best care, treatment failure is common and FSGS is causal in a significant proportion of end stage renal disease. Thus, an unmet need exists for novel disease modifying treatments for FSGS.

### SUMMARY OF THE INVENTION

The objective is solved by the subject of the present claims and as further described herein. It had been surprisingly found that the compound of the present invention significantly lowered urine protein/creatinine ratio (UPCR) and urine albumin/creatinine ratio (UACR) in animal studies thus demonstrating significant functional improvement and reduced proteinuria.

The invention provides a pharmaceutical preparation comprising clopidogrel, or a salt or solvate thereof, for use in the treatment of focal segmental glomerulosclerosis (FSGS).

Specifically, the pharmaceutical preparation described herein is used for the treatment of primary FSGS. Specifically, FSGS is not related to and is not caused by diabetes.

According to an embodiment of the invention, the pharmaceutical preparation is a medicinal product or a drug product, comprising clopidogrel, and a pharmaceutically acceptable carrier.

Specifically, the pharmaceutical preparation described herein is administered systemically.

According to an embodiment, clopidogrel used in the preparation described herein comprises the structure

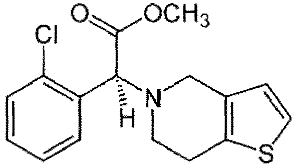

Specifically, the preparation described herein comprises about 10 to 400 mg clopidogrel.

According to a further embodiment, pharmaceutical preparation described herein is formulated for systemic administration, preferably for intravenous, intramuscular, subcutaneous, intradermal, transdermal, or oral administration.

According to a further embodiment, the pharmaceutical preparation described herein is administered to the subject as a spray, a powder, a gel, an ointment, a cream, a foam, or a liquid solution, a lotion, a gargle solution, an aerosolized powder, an aerosolized liquid formulation, granules, capsules, drops, tablet, syrup, lozenge, or a preparation for infusion or injection.

According to the invention the pharmaceutical preparation is for use in a subject is treated who suffers from FSGS or is at risk of developing FSGS.

In a further embodiment, the pharmaceutical preparation described herein is applied in an effective amount into a subject suffering from FSGS or being at risk of developing FSGS.

Specifically, the preparation is administered as the sole substance, or wherein treatment or preparation is combined with a further treatment or preparation with one or more active substances.

More specifically, the preparation is administered in combination with an active agent selected from the group consisting of antiviral drugs, anticoagulants, immune modulators, antibody preparations from human sources, monoclonal antibodies, intensive care medications, antihypertensive agents, statins, vasodilators, steroids, cytotoxic drugs, diuretics, non-steroidal anti-inflammatory drugs (NSAIDs), cholesterol or triglyceride reducing agents.

### FIGURES

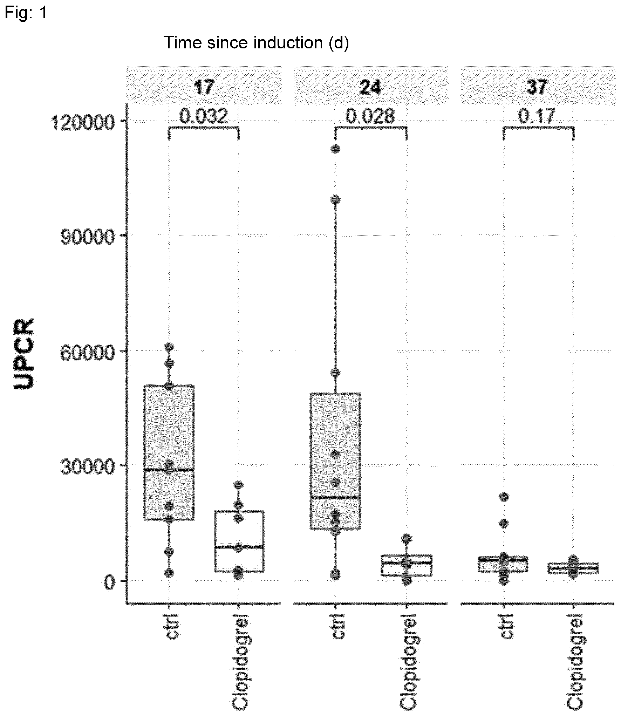

Figure 1: FSGS was induced with Adriamycin (ADR) and evaluated regarding proteinuria at the indicated times (in days) after induction. UPCR = urinary protein creatinine ratio - a marker for proteinuria and kidney damage; boxes marked "ctrl" = control mice with FSGS; boxes marked "Clopidogrel" = mice with FSGS, treated with Clopidogrel; Boxplots indicate the median (midline), inter-quartile range (box) and 95% confidence intervals (whiskers) in addition to individual data points (scatter). P-values of t-tests are indicated above the data for each time point.

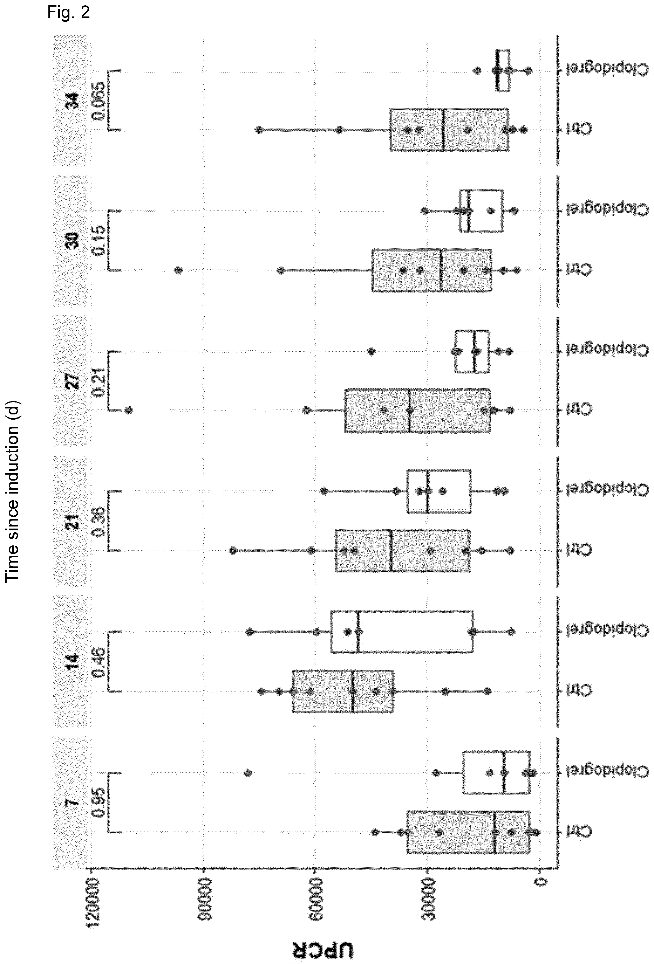

Figure 2: In an independent experiment FSGS was induced with Adriamycin (ADR) and evaluated regarding proteinuria at the indicated times (in days) after induction. UPCR = urinary protein creatinine ratio - a marker for proteinuria and kidney damage; boxes marked "ctrl" = control mice with FSGS; boxes marked "Clopidogrel" = mice with FSGS, treated with Clopidogrel; Boxplots indicate the median (midline), inter-quartile range (box) and 95% confidence intervals (whiskers) in addition to individual data points (scatter). P-values of t-tests are indicated above the data for each time point.

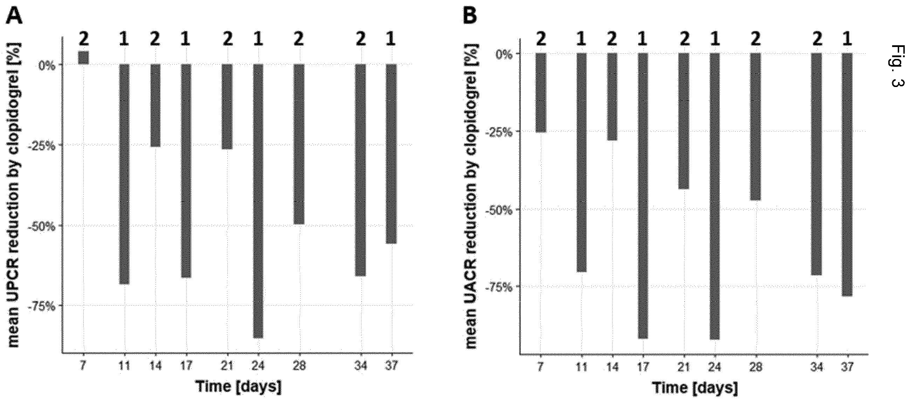

Figure 3: The mean reduction of UPCR (panel A) and UACR (panel B) due to clopidogrel treatment is indicated over time. Numbers on top indicate time points used in the first and second experiment respectively.

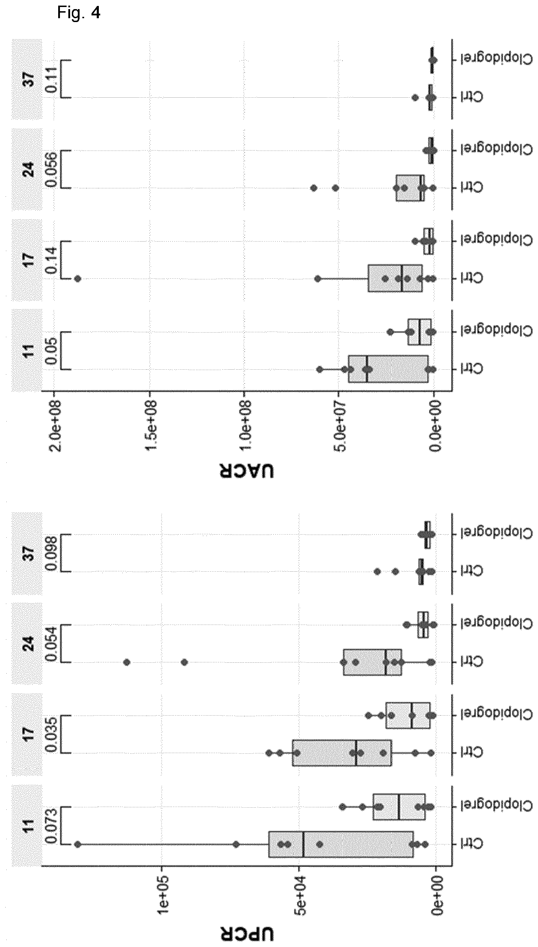

Figure 4: Experiment 1. UPCRs (left panel) and UACR (right panel) values of all individual urines of control and Clopidogrel treated animals after tail vein injection. p-values were calculated by Student's t-test.

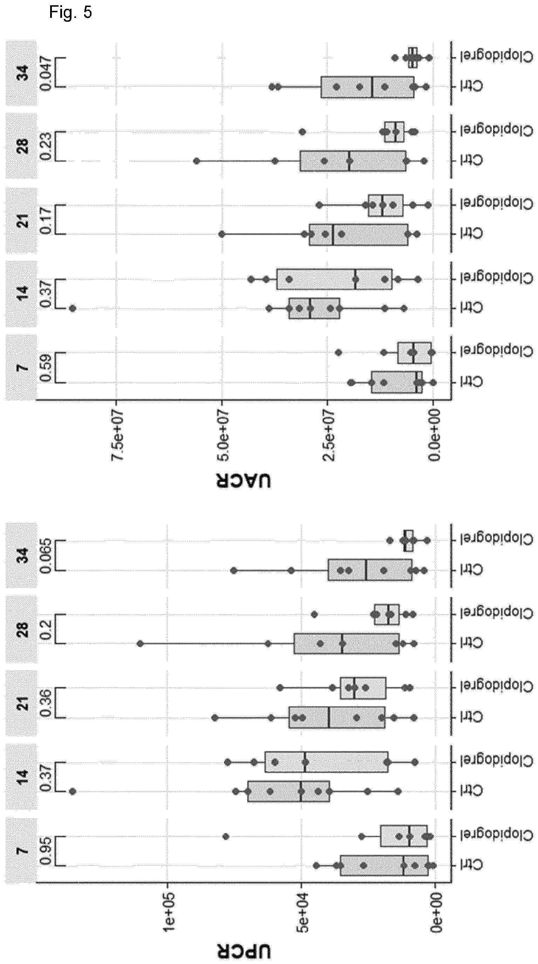

Figure 5: Experiment 2. UPCRs (left panel) and UACR (right panel) values of all individual urines of control and Clopidogrel treated animals after tail vein injection. p-values were calculated by Student's t-test.

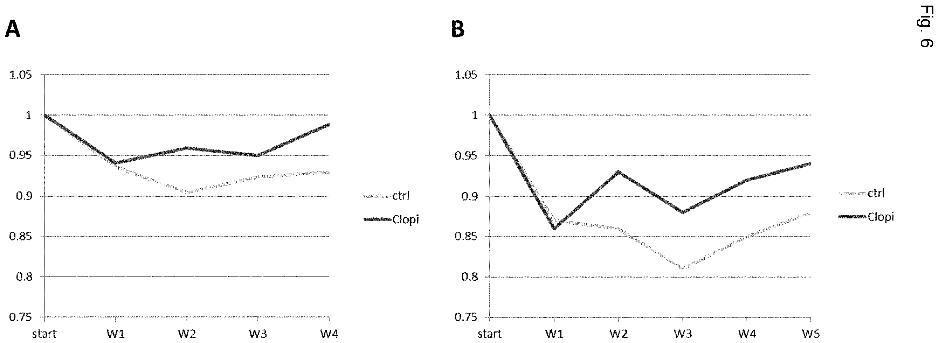

Figure 6: Weight change of the mice was measured weekly and is depicted as fold-change of the starting weight. (A) Experiment 1. (B) Experiment 2.

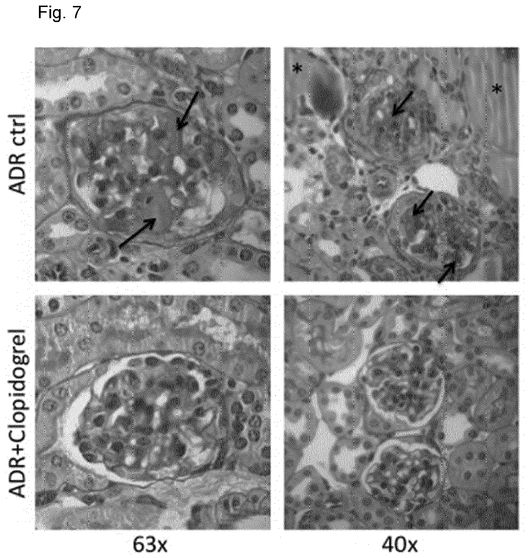

Figure 7: histological assessment with high magnification (63x, 40x). Representative PAS stainings of adriamycin nephropathy control animals (upper panels) and Clopidogrel-treated animals (lower panels) are shown. Black arrows indicate selected sclerotic areas. Black asterisks indicate protein casts representing to tubular ectasia.

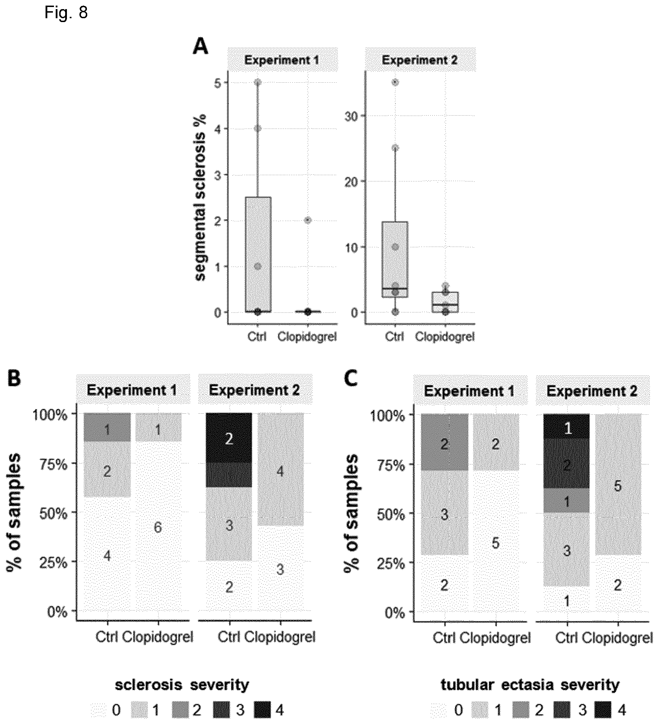

Figure 8: Histopathological effects of Clopidogrel therapy. (A) Average values of percentage of sclerotic glomeruli in two independent experiments given as box plots depicting all individual values. (B&C) 100%-stacked column chart depicting the abundance of the indicated groups depending on the relative amount of sclerotic glomeruli (B) or the severity of tubular ectasia (C). Numbers in the bar stacked charts represent the observation count (n). Segmental sclerosis scores: 0: n.d., 1: 1-4%, 2: 5-9%, 3: 10-19%, 4: >=20%. Tubular ectasia scores: 0: none, 1: mild, 2: moderate, 3: strong, 4: very strong.

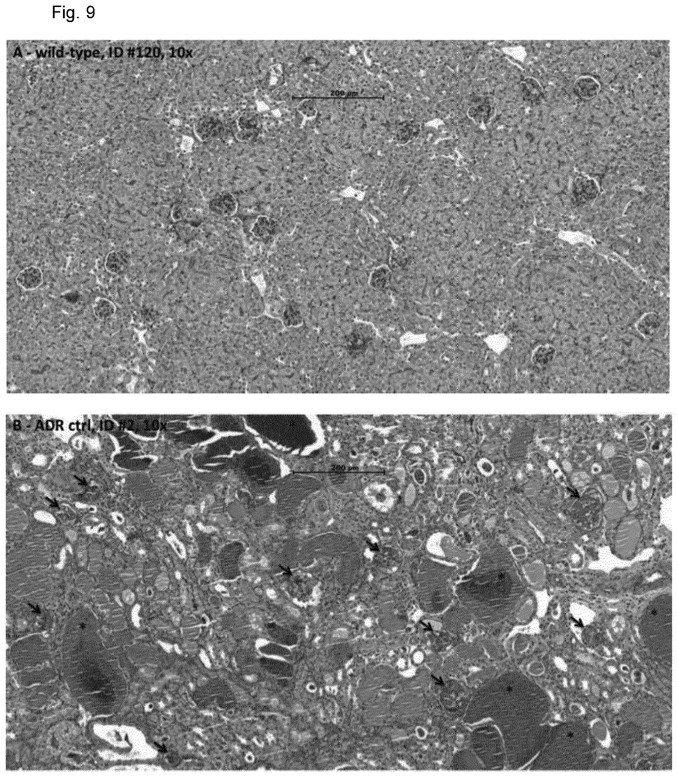

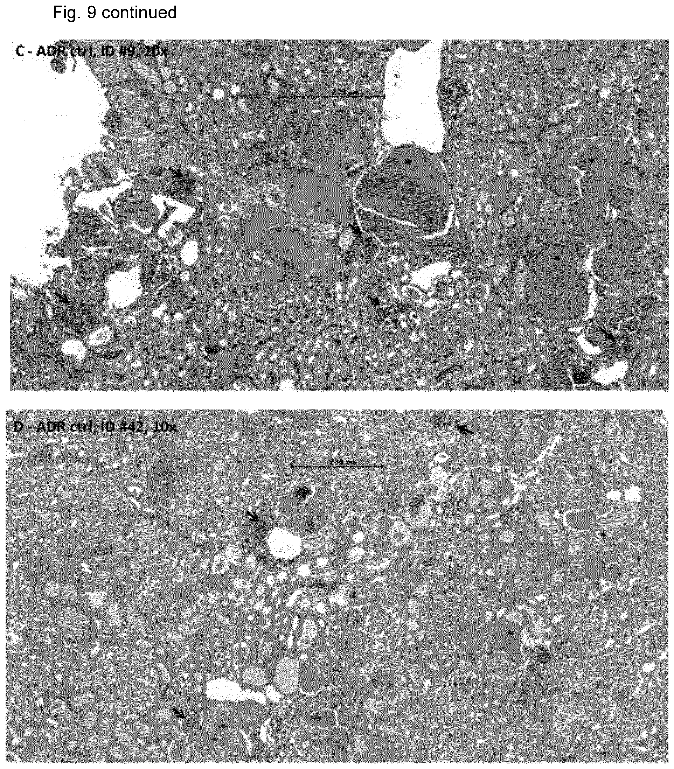

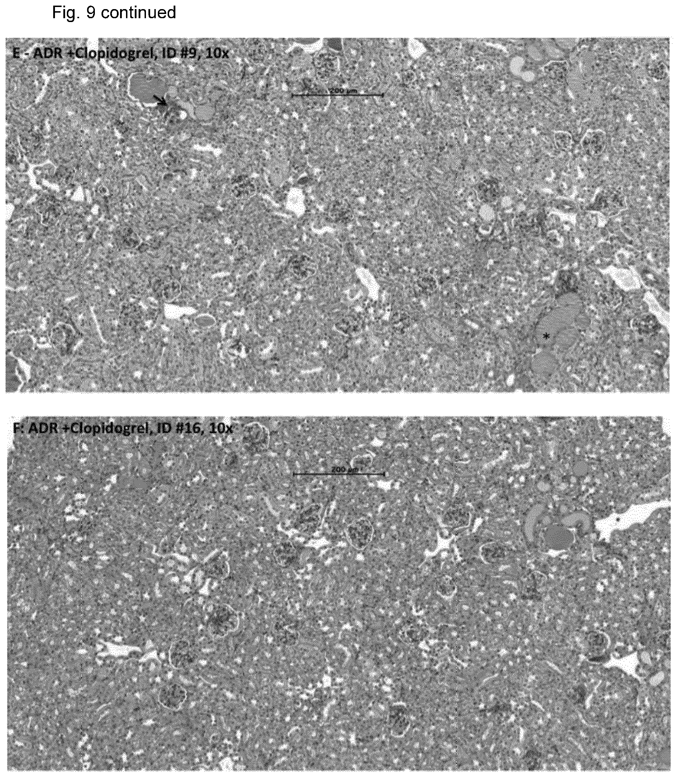

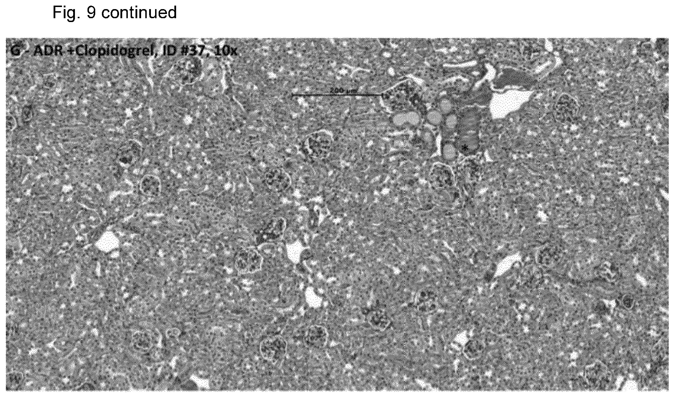

Figure 9: histological assessment of Clopidogrel therapy. Overview PAS stainings of (A) wild-type, (B-D) three Adriamycin nephropathy control animals, (E-G) three Adriamycin nephropathy treated with Clopidogrel are shown. Black arrows indicate selected sclerotic glomeruli. Black asterisks indicate selected protein casts due to tubular ectasia and a failure in the filtration process.

### DETAILED DESCRIPTION

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "about" as used herein refers to the same value or a value differing by +/-10% or +/-5% of the given value.

Clopidogrel as described herein may be used as a "physiologically acceptable salt". The choice of salt is determined primarily by how acid or basic the chemical is (the pH), the safety of the ionized form, the intended use of the drug, how the drug is given (for example, by mouth, injection, or on the skin), and the type of dosage form (such as tablet, capsule, or liquid).

Exemplary salts which are physiologically acceptable are sodium salts. However, it is also possible to employ, in place of the sodium salts, other physiologically acceptable salts, e.g., other alkali metal salts, alkaline earth metal salts, ammonium salts and substituted ammonium salts. Specific examples are potassium, lithium, calcium, aluminum and iron salts. Preferred substituted ammonium salts are those derived, for example, from lower mono-, di-, or trialkylamines, or mono-, di- and trialkanolamines. The free amino acids per se can also be used. Specific examples are ethylamine, ethylenediamine, diethylamine, or triethylamine salts.

The term "subject" or "patient" as used herein refers to a human being, or a non-human mammal, such as a dog, cat, horse, camelids, cattle or pig, suffering from FSGS or being at risk of developing FSGS. Specifically, it is a human being.

A subject "at risk" of developing FSGS may or may not have detectable disease or symptoms of disease, and may or may not have displayed detectable disease or symptoms of disease prior to the treatment methods described herein. "At risk" denotes that a subject has one or more risk factors, which are measurable parameters that correlate with development of FSGS, as described herein and known in the art. A subject having one or more of these risk factors has a higher probability of developing FSGS than a subject without one or more of these risk factor(s).

A "subject" can be a "patient". A "patient," refers to a "subject" who is under the care of a treating physician. In another embodiment, the patient is a subject who has not been diagnosed with FSGS. In yet other embodiments, the patient is a subject who has been diagnosed with FSGS but has not had any treatment to address the FSGS.

The term "non-diabetic subject" refers to a subject not suffering from Diabetes mellitus.

The term "pharmaceutically acceptable" also referred to as "pharmacologically acceptable" means compatible with the treatment of animals, in particular, humans. The term pharmacologically acceptable salt also includes both pharmacologically acceptable acid addition salts and pharmacologically acceptable basic addition salts.

The term" pharmacologically acceptable acid addition salt" as used herein means any non-toxic organic or inorganic salt of any base compound of the disclosure, or any of its intermediates. Basic compounds of the disclosure that may form an acid addition salt include, for example, compounds that contain a basic nitrogen atom. Illustrative inorganic acids which form suitable salts include hydrochloric, hydrobromic, sulfuric and phosphoric acids, as well as metal salts such as sodium monohydrogen orthophosphate and potassium hydrogen sulfate. Illustrative organic acids that form suitable salts include mono-, di-, and tricarboxylic acids such as glycolic, lactic, pyruvic, malonic, succinic, glutaric, fumaric, malic, tartaric, citric, ascorbic, maleic, benzoic, phenylacetic, cinnamic and salicylic acids, as well as sulfonic acids, such as p-toluene sulfonic and methanesulfonic acids. Either mono-, di- or triacid salts can be formed, and such salts may exist in either a hydrated, solvated or substantially anhydrous form. In general, the acid addition salts of the compounds of the disclosure are more soluble in water and various hydrophilic organic solvents, and generally demonstrate higher melting points in comparison to their free base forms. The selection of the appropriate salt will be known to one skilled in the art. Other non-pharmacologically acceptable acid addition salts, e.g. oxalates, may be used, for example, in the isolation of the compounds of the disclosure, for laboratory use, or for subsequent conversion to a pharmacologically acceptable acid addition salt. Specifically, clopidogrel may be present as clopidogrel hydrogen sulfate salt (clopidogrel bisulfate), sulfonate, or besylate salt. Clopidogrel may also be present as inclusion complex with a cyclodextrin, such as β-cyclodextrin, or a derivative thereof.

Clopidogrel, ((S)-Methyl-(2-chlorphenyl)-2-(6,7-dihydro-4H-thieno[3,2-c]pyridin-5-yl)acetate, (+)-Methyl-(2-chlorphenyl)-2-(6,7-dihydro-4H-thieno[3,2-c]pyridin-5-yl) acetate) as used herein has the structure 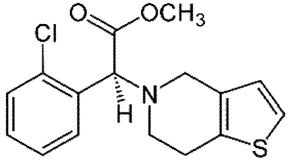 and is a prodrug of a platelet inhibitor used to reduce the risk of myocardial infarction and stroke. Clopidogrel is metabolized to its active form by carboxylesterase-1. The active form is a platelet inhibitor that irreversibly binds to P2Y12 ADP receptors on platelets. This binding prevents ADP binding to P2Y12 receptors, activation of the glycoprotein GPIIb/IIIa complex, and platelet aggregation.

In addition to its platelet aggregation inhibitor function, clopidogrel can also exert anti-inflammatory effects that can have beneficial impact on FSGS disease development and/or progression in addition to the surprising effect of UPCR and UACR lowering as described herein. Clopidogrel can interfere with TNF by activating the AMPK/Nrf2 axis (Yang H. et al., 2016). The impact of TNF-a on FSGS and podocyte injury is also described (Chen A. et al., 2020, Chung CF. et al., 2019, Pedigo C.E. et al., 2016), as is the importance of Nrf2 and AMPK for podocyte function (Yang SM., et al., 2013, Tsai PY. et al., 2011, Rogacka D. et al., 2020). Clopidogrel was shown to lower levels of TNF (Solheim S. et al., 2006) and also SERPINE1 (Sakata T. et al., 2011), two proteins linked to FSGS progression. Clopidogrel was also reported to induce NFE2L2 (NRF2), a factor with renoprotective potential that could contribute to alleviating FSGS disease progression (Yang H. et al., 2016). High IL10 levels on the other hand were shown to be associated with low clopidogrel responsiveness (Osmancik P. et al., 2012). VASP phosphorylation assay is used to measure clopidogrel response (Siller-Matula J.M. et al., 2010; Cayla G. et al., 2008). The vasodilator stimulated phosphoprotein (VASP) was shown to be phosphorylated in response to relapse plasma from ten consecutively tested patients, and not in response to paired remission plasma or non-FSGS controls. The phosphorylation signal is absent in human podocytes carrying a pathological podocin mutation (Harris J.J. et al., 2013).

Clopidogrel is sold under the trade names Clopilet, Plavix and Zyllt.

Clopidogrel comprises about 10 to 400 mg clopidogrel per dose. Specifically, it can comprise 10, 20, 30, 40, 50, 60, 70, 75, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400 mg clopidogrel. It may be administered continuously at the same dose or as bolar administration at high dose, such as about 300 mg and further as maintenance low dose of about 70 mg.

Particularly, in the pharmaceutical preparation of the present invention, a daily dosage of clopidogrel or pharmaceutically acceptable salts thereof is 10 to 400 mg, specifically 50 to 300 mg based on adults. However, the scope of the present invention is not limited to the dosage.

FSGS is a distinct clinico-pathological medical condition characterized by focal and segmental sclerosis in the kidney glomerulus and by podocyte foot process effacement (D'Agati V., 2003; Rosenberg A.Z. and Kopp J.B., 2017, Bose B. and Cattran D., 2014). The pathophysiology of FSGS can origin within the glomerulus ("primary FSGS") or be secondary to other reasons (e.g. hypertension; D'Agati V., 2003, Rosenberg A.Z. and Kopp J.B., 2017, Bose B. and Cattran D., 2014). FSGS frequently leads to nephrotic syndrome characterised by proteinuria, hypoalbuminuria, hyperlipidemia and edema (D'Agati V., 2003; Rosenberg A.Z. and Kopp J.B., 2017, Bose B. and Cattran D., 2014). For a high number of cases the exact cause of FSGS is still elusive (idiopathic; D'Agati V., 2003; Rosenberg A.Z. and Kopp J.B., 2017, Bose B. and Cattran D., 2014). Known etiologies for FSGS are heterogeneous and include gene mutations, drugs, viruses, hypertension, and circulating factors - but not diabetes (D'Agati V., 2003; Rosenberg A.Z. and Kopp J.B., 2017, Bose B. and Cattran D., 2014). Diabetic nephropathy - including diabetic glomerulosclerosis - represents the chronic loss of kidney function as a result of the pathophysiology of Diabetes mellitus (Qi et al., 2017).

Podocytes are atypical epithelial cells in the Bowman capsule in kidneys that wrap around capillaries of the glomerulus.

As used herein, FSGS is a scarring of glomeruli (sclerosis) and a damage to podocytes, specifically triggered by an endogenous factor that recognizes the podocyte as its target (podocytopathy, idiopathic FSGS), or caused by an identifiable cause that directly or indirectly injures podocytes (primary or secondary FSGS), such as a genetic disorder, hypertension, inflammation, deregulation of proliferation, or mechanical stress.

The distinctive morphological picture of primary FSGS is a diffuse podocytopathy (+ at least one FSGS lesion) clinically dominated by a nephrotic syndrome, The diffuse podocyte toxicity involves almost all podocytes, with more than 50% of foot process effacement at the electron microscopy. A massive loss of albumin in the urine as well as hypoalbuminemia are commonly observed but are unusual in secondary forms even if nephrotic range proteinuria is present. Microvillous transformation, cytoplasm shedding, increased density of actin cytoskeleton on effaced foot processes, increased number of lysosomes and auto-phagocytic bodies are common features. Due to structural reorganization the risk of detachment of podocytes from the glomerular basement membrane (GBM) is increased, leaving wide areas of bare GBM. These "sticky" areas can produce adhesions, after which the deposition of hyaline material and mesangial matrix expansion progressively narrows the capillary lumen, until the affected segment is obstructed and sclerosed. Reversible glomerular prolapses into the proximal tubule are occasionally observed, and are the expression of an acute enlargement of the tuft and predictive of a 'tip lesion' (Angioi A., and Pani, A., 2016).

The most aggressive form of FSGS is collapsing glomerulopathy, histologically shown by foot process effacement, segmental to global collapse of the capillary tuft, surrounded by a crown of hyperplastic podocytes (Angioi A., and Pani, A., 2016).

The symptoms of FSGS are also heavy proteinuria with optional biopsy confirmation of FSGS with glomerulosclerosis, glomerulonephritis (e.g. membranoproliferative glomerulonephritis (MPGN), IgA glomerulonephritis), nephrotic syndrome (hypoalbuminuria, hyperlipidemia, edema), progressive renal failure, glomerular lesions on histopathology, specifically as classified according to Haas M. et al., 2013, and podocyte fusion and injury.

FSGS can be diagnosed by methods well known in the art, such as, but not limited to determination of urinary protein /creatinine ratio (UPCR), urinary albumin / creatinine ratio (UACR), light microscopy of kidney biopsy, e.g. glomerular size, histologic variant of FSGS, microcystic tubular changes, and tubular hypertrophy; immunofluorescence, e.g. to rule out other primary glomerulopathies; and electron microscopy, e.g. extent of podocyte foot process effacement, podocyte microvillous transformation, and tubuloreticular inclusions.

Specifically, FSGS as used herein refers to non-diabetic renal disease. Specifically, diabetic nephropathy caused by Diabetes mellitus is excluded from the use of clopidogrel for the treatment of FSGS. Non-diabetic renal disease and diabetic renal disease can be confirmed and distinguished by methods known in the art such as biopsy.

In diabetes patients, nodular lesions and diffuse lesions are often observed. Classification methods for diabetic nephropathies are well known by the skilled person and are described by Qi C. et al., 2017, Tervaert T.W.C. et al., 2010, and Fioretto P. et al., 1996. Fioretto classification includes tubular, interstitial, and vascular lesions and divided diabetic nephropathies into 3 categories according to the pathological changes under light microscope: C1, normal/near normal; C2, typical diabetic nephropathy with predominantly glomerular changes; and C3, atypical patterns of injury, associated with disproportionately damage including tubulointerstitial or arteriolar hyalinosis and with absent or only mild diabetic glomerular changes. Tervaert pathological classification divides diabetic nephropathies into four classifications according to glomerular lesions, along with a separate scoring system for tubular, interstitial, and vascular lesions. Specifically, patients suffering from diabetes, specifically patients who are suffering from diabetes for more than 2 years, are excluded from being treated by clopidogrel as described herein.

Diabetic related nephropathies include diabetic retinopathy, diabetic nephropathy, and diabetes-related glomerulosclerosis. The use of the term "FSGS" covers native FSGS, primary FSGS as well as recurrent FSGS, but specifically excluding diabetic FSGS,

Glomerulonephritis describes the inflammation of the membrane tissue in the kidney that serves as a filter, separating wastes and extra fluid from the blood.

MPGN is a form of glomerulonephritis caused by an abnormal immune response. Deposits of antibodies build up in a part of the kidneys called the glomerular basement membrane.

Glomerulosclerosis describes the scarring or hardening of the tiny blood vessels within the kidney. Although glomerulonephritis and glomerulosclerosis have different causes, they can both lead to kidney failure.

Nephrotic syndrome is a kidney disorder that causes the body to pass too much protein in urine. Nephrotic syndrome is usually caused by damage to the clusters of small blood vessels in kidneys.

As used herein, recurrent FSGS (rFSGS), or recurrence of FSGS is defined by heavy proteinuria with optional biopsy confirmation of FSGS with glomerular sclerosis and podocyte fusion and injury without evidence of acute rejection, glomerulitis or allograft glomerulopathy. As used herein, a recurrent FSGS (rFSGS) subject or patient is defined as someone who had FSGS prior to kidney transplant and then developed a recurrence of FSGS (rFSGS) following kidney transplant.

As used herein, a non-recurrent FSGS (nrFSGS) subject or patient is defined as someone has FSGS prior to kidney transplant but does not develop FSGS following kidney transplant.

As used herein, a native FSGS (nFSGS) subject or patient is defined as someone who has FSGS (heavy proteinuria with optional biopsy confirmation of FSGS with glomerular sclerosis and podocyte fusion and injury) in his own kidney prior to transplant.

The pharmaceutical preparation can be a medicinal product or a drug product, comprising clopidogrel, and a pharmaceutically acceptable carrier. The preparation described herein can also be used as food supplement.

Herein provided is also a method for treatment of FSGS wherein the pharmaceutical preparation comprises clopidogrel in an effective amount.

Specifically, Clopidogrel or a pharmaceutically acceptable salt or solvate thereof significantly reduces podocyte injury and podocytopathy in FSGS.

The term "effective amount" or "pharmaceutically effective amount" refers to that amount of compound that produces the desired effect for which it is administered (e.g., improvement in symptoms of FSGS, lessening the severity of FSGS or a symptom of FSGS, and/or reducing progression of FSGS or a symptom of FSGS). The exact amount of an effective dose will depend on the purpose of the treatment and will be ascertainable by one skilled in the art using known techniques (see, e.g., Lloyd V.A., 2016).

As used herein, the term "treatment" and its cognates refer to slowing or stopping disease progression. "Treatment" and its cognates as used herein, include, but are not limited to the following: complete or partial remission, lower risk of kidney failure (e.g. ESRD), and disease-related complications (e.g. edema, susceptibility to infections, or thrombo-embolic events). Improvements in or lessening the severity of any of these symptoms can be readily assessed according to methods and techniques known in the art or subsequently developed. Desirable effects of treatment include preventing the occurrence or recurrence of FSGS, specifically non-diabetic FSGS, or a condition or symptom thereof, alleviating a condition or symptom of FSGS, diminishing any direct or indirect pathological consequences of FSGS, decreasing the rate of FSGS progression or severity, and/or ameliorating or palliating the FSGS. In some embodiments, methods and compositions of the invention are used on patient sub-populations identified to be at risk of developing FSGS. In some cases, the methods and compositions of the invention are useful in attempts to delay development of FSGS.

In some embodiments, the compound of the pharmaceutical preparation described herein (i.e. clopidogrel), or a pharmaceutically acceptable salt or solvate thereof is not administered with any other therapeutic compound. In some embodiments, the compound is not administered with any other therapeutic compound, concurrently or sequentially. In some embodiments, the compound is administered alone.

In some embodiments, the method further comprises administering to the patient one or more additional therapeutic compound. In some embodiments, the one or more additional therapeutic compound is selected from one or more of an antiviral drug, an anticoagulant, an immune modulator, an antibody preparation from human sources, a monoclonal antibody, an intensive care medication, an antihypertensive agent, a statin, a vasodilator, an steroid, a cytotoxic drug, a diuretic, a non-steroidal anti-inflammatory drug (NSAID), a cholesterol or triglyceride reducing agent, PPAR agonists or phytopharmacological substances such as myristic acid.

The at least one pharmaceutically acceptable carrier, as used herein, includes any and all solvents, diluents, other liquid vehicles, dispersion aids, suspension aids, surface active agents, isotonic agents, thickening agents, emulsifying agents, preservatives, solid binders, and lubricants, as suited to the particular dosage form desired. Remington: The Science and Practice of Pharmacy, 22nd edition, 2013, Pharmaceutical Press, and Encyclopedia of Pharmaceutical Technology, 2004, Taylor & Francis, disclose various carriers used in formulating pharmaceutical compositions and known techniques for the preparation thereof. Except insofar as any conventional carrier is incompatible with the compounds of this disclosure, such as by producing any undesirable biological effect or otherwise interacting in a deleterious manner with any other component(s) of the pharmaceutical preparation, its use is contemplated to be within the scope of this disclosure. Non-limiting examples of suitable pharmaceutically acceptable carriers include, but are not limited to, ion exchangers, alumina, aluminum stearate, lecithin, serum proteins (such as human serum albumin), buffer substances (such as phosphates, glycine, sorbic acid, and potassium sorbate), partial glyceride mixtures of saturated vegetable fatty acids, water, salts, and electrolytes (such as protamine sulfate, disodium hydrogen phosphate, potassium hydrogen phosphate, sodium chloride, and zinc salts), colloidal silica, magnesium trisilicate, polyvinyl pyrrolidone, polyacrylates, waxes, polyethylene-polyoxypropylene-block polymers, wool fat, sugars (such as lactose, glucose and sucrose), starches (such as corn starch and potato starch), cellulose and its derivatives (such as sodium carboxymethyl cellulose, ethyl cellulose and cellulose acetate, microcrystalline cellulose, methyl cellulose, sodium carboxymethyl cellulose), lactose, dextrin, mannitol, white sugar, maize starch, pre-gelatinized starch, precipitated calcium carbonate and calcium hydrogen phosphate,, powdered tragacanth, malt, gelatin, talc, excipients (such as cocoa butter and suppository waxes), oils (such as peanut oil, cottonseed oil, safflower oil, sesame oil, olive oil, corn oil and soybean oil), glycols (such as propylene glycol and polyethylene glycol), esters (such as ethyl oleate and ethyl laurate), agar, buffering agents (such as magnesium hydroxide and aluminum hydroxide), alginic acid, pyrogen-free water, isotonic saline, Ringer's solution, ethyl alcohol, phosphate buffer solutions, non-toxic compatible lubricants (such as sodium lauryl sulfate and magnesium stearate), coloring agents, releasing agents, coating agents, sweetening agents, flavoring agents, perfuming agents, preservatives, and antioxidants or any mixtures and combinations thereof.

Solubility of clopidogrel or its salts may be increased by methods known in the art such as, but not limited by combination with non-aqueous agents such as polyol or glycerol, or polar solvents such as propylene glycol.

### EXAMPLES

The foregoing description will be more fully understood with reference to the following examples. Such examples are, however, merely representative of methods of practicing one or more embodiments of the present invention and should not be read as limiting the scope of invention.

### Example 1

### In vivo Testing

For biological testing of clopidogrel, the accepted mouse model of FSGS was chosen, i.e. the adriamycin induced nephropathy. Adriamycin nephropathy is the rodent state-of-the-art model of human primary focal segmental glomerulosclerosis (FSGS) and is characterized by podocyte injury followed by glomerulosclerosis, tubulointerstitial inflammation and fibrosis (Da Sacco S. et al., 2014, Lee V.W. and Harris D.C., 2011)) and was therefore used for demonstrating the therapeutic effect of clopidogrel in FSGS.

All animal experiments and handling were in accordance with the Austrian law for protection of animals and approved by the animal ethics committee of the Austrian ministry for science and research.

Adriamycin-dependent nephropathy (active compound: doxorubicin) was induced by tail vein injection accepted doses of approx. 9 mg/kg mouse in 10 weeks old BALB/c mice. Control mice were injected with 0.9% saline. At least 6 mice were analyzed per group. Mice were monitored daily for signs of pain, altered movement, or reduced food uptake and sacrificed by cervical dislocation. Tested drugs were administered per oral administration via pre-mixed chow at drug doses based on literature research.

As a further model, or miR-193a-overexpressing mice which suffer from FSGS due to suppression of Wilms' tumor 1 (WT1) gene could be used. (Gebeshuber et al., 2013).

### Results

Urinary albumin levels were assessed by ELISA, urinary total protein and urinary creatinine levels were measured on the Roche Cobas system, using routine laboratory kits. Urinary protein/creatinine (UPCR) ratio and urinary albumin/creatinine ratio (UACR) were assessed at different time points. Weight and physical fitness scores were monitored on a daily basis. At the end of the experiment, tissue sections, plasma and urine samples were collected and stored until further analysis. For successful drug candidates significantly lowered UPCR and UACR values were found, demonstrating a functional improvement and reduced proteinuria.

Repeated measures ANOVA across timepoints was calculated using the R statistical software framework. Effects for the drug administration (Clopidogrel) and timepoint (in days) were significant with p-values of 0.009 (drug) and 0.023 (timepoint), respectively. The p-value for the interaction (drug:timepoint) was 0.788 (see Figure 2).

In Figure 1, it is shown that FSGS was induced with Adriamycin (ADR) and evaluated regarding proteinuria at the indicated times (in days) after induction. UPCR = urinary protein creatinine ratio; UACR = urinary albumin creatinine ratio - both markers for proteinuria and kidney damage; Grey boxes (ctrl) = control mice with FSGS; boxes marked "Clopidogrel" = mice with FSGS, treated with Clopidogrel; Boxplots indicate the median (midline), inter-quartile range (box) and 95% confidence intervals (whiskers) in addition to individual data points (scatter). P-values of t-tests are indicated above the data for each time point. UPCR in both Ctrl and Clopidogrel treated mice at 37 days after start is reduced to similar levels because mice could recover from Adriamycin induced FSGS in this experiment.

In Figure 2 the following is shown: in an independent experiment FSGS was induced with Adriamycin (ADR) and evaluated regarding proteinuria at the indicated times (in days) after induction. UPCR = urinary protein creatinine ratio - a marker for proteinuria and kidney damage; Grey boxes (ctrl) = control mice with FSGS; boxes marked "Clopidogrel" = mice with FSGS, treated with Clopidogrel; Boxplots indicate the median (midline), inter-quartile range (box) and 95% confidence intervals (whiskers) in addition to individual data points (scatter). P-values of t-tests are indicated above the data for each time point. Results for repeated measures ANOVA to test clopidogrel across timepoints are shown above

### Example 2

### In vivo Testing

For biological testing of clopidogrel, the accepted mouse model of FSGS was chosen, i.e. the adriamycin induced nephropathy. Adriamycin nephropathy is the rodent state-of-the-art model of human primary focal segmental glomerulosclerosis (FSGS) and is characterized by podocyte injury followed by glomerulosclerosis, tubulointerstitial inflammation and fibrosis (Da Sacco S. et al., 2014, Lee V.W. and Harris D.C., 2011) and was therefore used for demonstrating the therapeutic effect of clopidogrel in FSGS.

All animal experiments and handling were in accordance with the Austrian law for protection of animals and approved by the animal ethics committee of the Austrian ministry for science and research.

### Materials & Methods

Adriamycin (Doxorubicin; Sigma, D1515-10mg) was diluted in ddH₂O. To induce FSGS, a dose of 10.5mg/kg mouse was injected i.v. into the tail vein using 27G needles, i. e. a total volume of 200 µl volume per 25 g mouse.

For tail vein injection, mice were anaesthetized using Ketanest (Pfizer, 25mg/ml vials) and Rompun (Bayer, 20mg/ml injection solution). A final mix of 12.5mg/ml Ketanest and 0.25% Rompun in 0.9% NaCl solution was prepared and 200µl/25g mouse were injected. Clopidogrel bisulfate (Sigma, PHR1431-1G) was used for therapy experiments.

### Animals/Animal maintenance

### Mice and FSGS model

Female Balb/c mice were obtained from the animal breeding facility of the Medical University (Abteilung für Labortierkunde, Himberg, Austria). 12-16 weeks old animals with a weight >20g were used for the experiments and adjusted for weight and age. Animals were allowed acclimatization of at least 7 days. Animals were marked by ear or toe clip codes to allow identification. Adriamycin nephropathy in Balb/c mice is the most commonly used animal model for preclinical FSGS studies and shares the central features with human FSGS.

### Diet

Standard commercial chow from ssniff (ssniff Spezialdiäten GmbH, Soest, Germany) contained crude protein (19%), crude fat (3.3%), crude fiber (4.9%), crude ash (6.4%), starch (36.5%), sugar (4.7%). Control group animals were fed standard chow, test group animals standard chow + Clopidogrel (200mg/kg) produced by ssniff. All animals were allowed to eat *ad libitum* assuming a daily average uptake of ~4 grams/mouse (according to animal care guideline of the Johns Hopkins University, Baltimore, MD, USA; http://web.jhu.edu/animalcare/ procedures/mouse.html), i. e. ~25-30 mg clopidogrel/kg mouse/day (Halim et al., 2019).

### Housing

The animals were kept in macrolon cages 375mm x 215mm x 150mm at standard conditions (23±1°C, 55±10% humidity, 12h day-night-cycle) and daily checked. Chow and water were provided *ad libitum.*

### Urine collection and measurements

Spot urine was collected every week and Creatinine and protein levels were measured.

Urinary albumin was measured by ELISA. 96-well plates were coated over night at 4°C with 100µl antibody solution Anti Mouse Serum Albumin antibody (Abcam ab34807, Lot GR3242102-4; diluted 1 :2,000) in coating buffer (3.7g NaHCOs; 0,64g Na₂CO₃; 1L distilled water). The remaining procedure was performed at room temperature. Wells were washed 3x with HBSS (Sigma H8264-500ML) +0,05% Tween-20 (Bio-Rad, #1706531) and blocked in ELISA/ELISPOT Diluent (eBioscience, 00-4202-56) and washed again for 3x. Samples and standard curve were both diluted in ELISA/ELISPOT diluent were incubated for 2h, followed by 3 wash steps with HBSS+0,05% Tween-20, incubated with HRP antibody Anti Mouse Serum Albumin antibody (Abcam ab19195, Lot GR3242102-4; diluted 1:100,000) for 1h, followed by 5 wash steps. Reaction was developed with TMB One Solution (Promega, G7431) and stopped with 2N H₂SO₄. Standard curves were generated with Mouse Albumin (Merck/Sigma, 126674-25MG). 450nm absorbance was measured on an Epoch Microplate reader (BioTek) using Gen5 1.10 software.

### Histology

At the end of the experiment kidneys were removed, embedded in paraffin and PAS (Periodic acid-Schiff) staining was performed. Histology was thoroughly evaluated and the amount of sclerotic glomeruli and protein casts was quantified.

### Statistics

R version 4.0.2 was used for statistical analysis. Student's t-test was used for comparing UPCR and UACR values between samples from treated and control mice for each individual time point and to assess differences in histological parameters. Repeated measures ANOVA was performed to analyse the drug's impact on UPCR and UACR over time using the respective functions in the rstatix package (V0.6.0). ggplot2 (V3.3.2) and ggpubr (V0.4.0) packages were used for generating the graphical visualizations.

### Experimental setup

In two independent experiments (Experiment 1 and 2), 34 female Balb/c mice were injected with 10.5mg/kg adriamycin into the tail vein (see table 2). 48 hours post injection the active group received standard chow + 200mg/kg Clopidogrel, while control mice stayed with standard chow. Spot urine was collected weekly, kidneys were harvested at sacrifice. 10.5mg/kg Adriamycin were injected into the tail vein. After 2 days Clopidogrel chow therapy was started. Urine was collected and weight was measured weekly. The experiments lasted about 5 weeks.

In the following Table 1, an overview of the mice that were used in the experiments is shown (* kidneys were harvested in 7 mice from each group; ** 1 animal was found dead in each group).

**Table 1**

| | Experiment 1 | | Experiment 2 | |
|---|---|---|---|---|
| | started | completed | started | completed |
| Control | 9 | 9* | 9 | 8** |
| Clopidogrel | 8 | 8* | 8 | 7** |

### Results

### Clopidogrel significantly reduces albuminuria and proteinuria

The determination of the urinary albumin to creatinine ratio (UACR) and the urinary protein to creatinine ratio (UPCR) revealed a marked amelioration with clopidogrel therapy. Fig. 3 depicts the percent reductions of the UACR and UPCR of two independent experiments. Fig. 4 depicts box plots and p-values of all individual collected urines of experiment 1.

Repeated measures ANOVA revealed a significant beneficial impact of clopidogrel treatment over time on outcome determined based on UPCR and UACR levels (p=0.000475 for UPCR and p=0.004 for UACR).

**ANOVA Table (type III tests) - UPCR**

| Effect | DFn | DFd | F | p | p<.05 | ges |
|---|---|---|---|---|---|---|
| **Experiment.drug** | 1 | 56 | 13.779 | **0.000475** | * | 0.197 |
| Timepoint.days | 3 | 56 | 3.191 | 0.030000 | * | 0.146 |
| Experiment.drug:Timepoint.days | 3 | 56 | 1.175 | 0.327000 | | 0.059 |

**ANOVA Table (type III tests) - UACR**

| Effect | DFn | DFd | F | p | p<.05 | ges |
|---|---|---|---|---|---|---|
| **Experiment.drug** | 1 | 57 | 9.007 | **0.004** | * | 0.136 |
| Timepoint.days | 3 | 57 | 2.041 | 0.118 | | 0.097 |
| Experiment.drug:Timepoint.days | 3 | 57 | 1.253 | 0.299 | | 0.06 |

Repeated measures ANOVA also showed the beneficial impact of clopidogrel treatment over time on outcome determined based on UPCR and UACR levels in the second independent experiment (Fig. 5) (p=0.029 for UPCR and p=0.009 for UACR).

**ANOVA Table (type III tests) - UPCR**

| | Effect | DFn | DFd | F | p | p<.05 | ges |
|---|---|---|---|---|---|---|---|
| 1 | Experiment.drug | 1 | 66 | 4.978 | **0.029** | * | 0.070 |
| 2 | Timepoint.days | 4 | 66 | 3.949 | 0.006 | * | 0.193 |
| 3 | Experiment.drug:Timepoint.days | 4 | 66 | 0.447 | 0.774 | | 0.026 |

**ANOVA Table (type III tests) - UACR**

| | Effect | DFn | DFd | F | p | p<.05 | ges |
|---|---|---|---|---|---|---|---|
| 1 | Experiment.drug | 1 | 66 | 7.175 | **0.009** | * | 0.098 |
| 2 | Timepoint.days | 4 | 66 | 4.418 | 0.003 | * | 0.211 |
| 3 | Experiment.drug:Timepoint.days | 4 | 66 | 0.289 | 0.884 | | 0.017 |

The beneficial impact of clopidogrel was thus confirmed in two independent experiments.

Taken both experiments together, clopidogrel reduced UPCR by 49% on average across all time points (repeated measures ANOVA p= 7.84e-05) and UACR by 61% on average across all time points (repeated measures ANOVA p=0.000162).

**ANOVA Table (type III tests) - UPCR**

| | Effect | DFn | DFd | F | p | p<.05 | ges |
|---|---|---|---|---|---|---|---|
| 1 | Experiment.drug | 1 | 122 | 16.710 | **7.84e-05** | * | 0.120 |
| 2 | Timepoint.days | 8 | 122 | 4.123 | 2.21e-04 | * | 0.213 |
| 3 | Experiment.drug:Timepoint.days | | 8 | 122 | 0.815 | 5.91e-01 | 0.051 |

**ANOVA Table (type III tests) - UACR**

| | Effect | DFn | DFd | F | p | p<.05 | ges |
|---|---|---|---|---|---|---|---|
| 1 | Experiment.drug | 1 | 123 | 15.147 | **0.000162** | * | 0.110 |
| 2 | Timepoint.days | 8 | 123 | 2.427 | 0.018000 | * | 0.136 |
| 3 | Experiment.drug:Timepoint.days | 8 | 123 | 1.076 | 0.384000 | | 0.065 |

The determination of UPCR and UACR therefore revealed a marked and significant protection of renal function with clopidogrel therapy.

### Overall health status - weight change:

Weight loss is a common symptom of kidney disease and also a consequence of the toxicity of Adriamycin. The weight change was evaluated weekly over the course of the experiments. We observed that Clopidogrel-treated mice suffered less weight loss as compared to control mice.

**ANOVA Table (type III tests) - Weight**

| | Effect | DFn | DFd | F | p | p<.05 | ges |
|---|---|---|---|---|---|---|---|
| 1 | Experiment.drug | 1 | 129 | 7.607 | **7.00e-03** | * | 0.056 |
| 2 | Timepoint.days | 8 | 129 | 6.752 | 2.25e-07 | * | 0.295 |
| 3 | Experiment.drug:Timepoint.days | 8 | 129 | 0.634 | 7.48e-01 | | 0.038 |

Overall, clopidogrel reduced ADR-associated weight loss by 48% (repeated measures ANOVA p=0.007).

### Histopathological assessment

Histopathological assessment confirmed that animals with adriamycin induced nephropathy show distinctive glomerular lesions and also striking tubular changes (see Figure 7 and Figure 9). Glomeruli are frequently affected by a mostly segmental (affecting only parts of the glomerular tuft) but sometimes also global sclerotic obsolescence of glomerular capillaries. These lesions may both in animal models and in humans result from podocyte injury and are diagnostic hallmarks of progressive disease. Tubular injury is present in untreated animals. Proximal tubuli are highly dilated and lined by flattened epithelial cells and contain homogenous protein casts. These tubular changes indicate tubular dysfunction and are non-specific and may be observed in primary tubulointerstitial disease (chronic interstitial nephritis) but can also occur along with severe glomerular injury. Pathogenic mechanisms might be obstruction of the urinary flow as downstream cause of tubular dilatation, but also glomerular injury especially when associated with high grade proteinuria which likely is causing the changes observed here.

While FSGS is initiated by glomerular damage, the ultimate cause of ESRD is a failure of the tubular reabsorption process. Fig. 7 and Fig. 9 demonstrate that the above-described changes are absent or at least much less pronounced with only very few glomeruli affected by segmental scleroses (as marked by arrows) and also very small and strikingly reduced tubular injury (as marked by asterisks on the compact appearing protein casts).

Fig. 8 represents the quantification of histopathological damage in both groups. The damage score, defined as the sum of the glomerular sclerosis severity score and the tubular ectasia severity score, depicts a significant amelioration of the disease after clopidogrel therapy of about 67%. Of note, Adriamycin nephropathy is characterized by a strong heterogeneity in glomerular sclerosis (Zhou et al. 2019; Xiong et al. 2020), which is also reflective of the human condition. Importantly, clopidogrel completely prevented severe disease - a major therapeutic goal - and in none of the samples a glomerular sclerosis index above 4% of all glomeruli was reached, while in the untreated control group the sclerosis index went up to 35% (Fig. 8A, B). Tubular ectasia, determined by protein cylinders in PAS staining, is an alternate measure to identify a failure in the renal filtration process and was also strongly ameliorated by Clopidogrel (Fig. 8C).

For morphological details of the analyzed sections and full field of view images, see Fig. 9 representing the 3 most strongly affected cases from each group.

Overall, clopidogrel reduced the histopathological damage by 67.9% (95% confidence interval -11.7% to -98.1%) from an average score of 2.67 to 0.86 (p=0.038).

The determination of sclerotic glomeruli and ectatic tubuli therefore revealed a marked and significant amelioration of histopathological damage with clopidogrel therapy.

In summary, clopidogrel treatment strongly improved the central read-out parameters for FSGS, namely UACR, UPCR and the severity of histopathological changes.

### Example 3

Electron microcopy is performed to classify the FSGS lesions in the animals with adriamycin induced nephropathy and confirm that these animals show that podocytes are affected by diffuse podocyte toxicity showing a high percentage of foot process effacement, podocyte microvillous transformation and tubuloreticular inclusions while animals treated with clopidogrel as described above have significantly reduced podocyte injury (podocytopathy).

### REFERENCES

Angioi A., and Pani A., FSGS: from pathogenesis to histological lesion, J. Nephrol. (2016), Nephrol., 29(4):517-23
Bose B., Cattran D., Glomerular Diseases: FSGS, 2014, Clin. J. Am.Soc.Nephrol., 9, 626-632
Cayla G. et al., Flow cytometric assessment of vasodilator-stimulated phosphoprotein: prognostic value of recurrent cardiovascular events after acute coronary syndromes, 2008, 101(11-12), 743-51
Chen A. et al., Soluble RARRES1 induces podocyte apoptosis to promote glomerular disease progression, 2020, J.Clin.lnvest, 130(10), 5523-5535
Chung CF. et al., Intrinsic tumor necrosis factor-α pathway is activated in a subset of patients with focal segmental glomerulosclerosis, 2019, PLoS One, 14(5):e0216426
Da Sacco S. et al., Renal Regeneration, Adriamycin Nephropathy, Dubois'Lupus Erythematosus and related Syndromes (9th edition), 2019
D'Agati V., Pathologic classification of focal segmental glomerulosclerosis, 2003, Semin Nephrol, 23(2), 117-34
Fioretto, P. et al., 1996, Patterns of renal injury in NIDDM patients with microalbuminuria, Diabetologia, vol. 39, no. 12, pp. 1569-1576
Gebeshuber CA, et al. Focal segmental glomerulosclerosis is induced by microRNA-193a and its downregulation of WT1, 2013, Nat Med., 19(4),481-487
Halim H., et al., Ticagrelor Induces Paraoxonase-1 (PON1) and Better Protects Hypercholesterolemic Mice against Atherosclerosis Compared to Clopidogrel, 2019, PloS One, 14 (6), e0218934. https://doi.org/10.1371/journal.pone.0218934.
Harris J.J. et al., Active proteases in nephrotic plasma lead to a podocin-dependent phosphorylation of VASP in podocytes via protease activated receptor-1, 2013, J Pathol., 229(5), 660-71
Lee V.W. and Harris D.C., Adriamycin nephropathy: A model of focal segmental glomerulosclerosis, Nephrology, 16, 2011, 30-38
Lloyd V.A. Jr., 2016, The Art, Science, and Technology of Pharmaceutical Compounding, 5th Edition, elSBN: 1-58212-263-6
Osmancik P. et al., High leukocyte count and interleukin-10 predict high on-treatment-platelet-reactivity in patients treated with clopidogrel, 2012, J Thromb Thrombolysis, 33(4), 349-54
Pedigo C.E. et al., Local TNF causes NFATc1-dependent cholesterol-mediated podocyte injury, 2016, J. Clin. Invest, 126(9), 3336-50
Peters H, et al. Platelet inhibition limits TGF-beta overexpression and matrix expansion after induction of anti-thy1 glomerulonephritis. Kidney Int. 2004, 65(6), 2238-2248.
Qi C. et al., Classification and differential diagnosis of diabetic nephropathy, Journal of Diabetes Research, 2016, ID8637138, 1-7.
Rogacka D. et al., Regulation of podocytes function by AMP-activated protein kinase, 2020, Arch Biochem Biophys, 692, 108541
Rosenberg A.Z. and Kopp, J.B., Focal segmental glomerulosclerosis, Clin J Am Soc Nephrol 12: 502-517, 2017
Sakata T. et al., Antiplatelet therapy effectively reduces plasma plasminogen activator inhibitor-1 levels, 2011, Artherosclerosis, 214(2), 490-1
Siller-Matula J.M. et al., Multiple electrode aggregometry predicts stent thrombosis better than the vasodilator-stimulated phosphoprotein phosphorylation assay, 2010, 8(2), 351-9
Solheim S et al., No difference in the effects of clopidogrel and aspirin on inflammatory markers in patients with coronary heart disease, 2006, Thromb. Haemost., 96(5), 660-4
Su X, et al., Effect of antiplatelet therapy on cardiovascular and kidney outcomes in patients with chronic kidney disease: a systematic review and meta-analysis. BMC Nephrol. 2019, 20(1). doi:10.1186/s12882-019-1499-3
Tervaert, T.W.C., et al., Pathologic classification of diabetic nephropathy, Journal of the American Society of Nephrology, 2010, vol.21, no.4, pp. 556-563
Tsai PY. et al., Antroquinonol reduces oxidative stress by enhancing the Nrf2 signaling pathway and inhibits inflammation and sclerosis in focal segmental glomerulosclerosis mice, 2011, Free Radic Biol Med, 50(11), 1503-16
Tu X. et al., Anti-inflammatory Renoprotective Effect of Clopidogrel and Irbesartan in Chronic Renal Injury, 2008, J.Am.Soc.Nephrol., 19(1), 77-83
Troyanov S, et al.; Toronto Glomerulonephritis Registry Group. Focal and segmental glomerulosclerosis: definition and relevance of a partial remission, 2005, J Am Soc Nephrol., 16(4),1061-1068
Xiong T., et al. Interleukin-9 Protects from Early Podocyte Injury and Progressive Glomerulosclerosis in Adriamycin-Induced Nephropathy. Kidney International 2020, 98 (3): 615-629.
Yang H. et al., Clopidogrel Protects Endothelium by Hindering TNFα-InducedVCAM-1 Expression through CaMKβ//AMPK/Nrf2 Pathway, 2016, J Diabetes Res. 1-10
Yang SM., et al., Citral is renoprotective for focal segmental glomerulosclerosis by inhibiting oxidative stress and apoptosis and activating Nrf2 pathway in mice, 2013, PLoS One, 8(9), e74871
Zhou D., et al., Sonic Hedgehog Connects Podocyte Injury to Mesangial Activation and Glomerulosclerosis, 2019, JCI Insight, 4 (22). https://doi.org/10.1172/jci.insight.130515

## Claims

1. Pharmaceutical preparation comprising clopidogrel, or a salt or solvate thereof, for use in the treatment of a non-diabetic subject suffering from focal segmental glomerulosclerosis (FSGS).

2. The pharmaceutical preparation for use according to claim 1, wherein clopidogrel is present as single active agent.

3. The pharmaceutical preparation for use according to claim 1 or 2, wherein FSGS is a primary FSGS.

4. The pharmaceutical preparation for use according to any one of claims 1 to 3, wherein the pharmaceutical preparation is a medicinal product or a drug product, comprising clopidogrel, and a pharmaceutically acceptable carrier.

5. The pharmaceutical preparation for use according to any one of claims 1 to 4, wherein the preparation is administered systemically.

6. The pharmaceutical preparation for use according to any one of claims 1 to 5, wherein clopidogrel comprises the structure

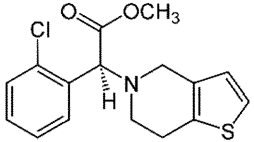

7. The pharmaceutical preparation for use according to claim 6, wherein the preparation comprises about 10 to 400 mg clopidogrel.

8. The pharmaceutical preparation for use according to any one of claims 1 to 7, wherein said pharmaceutical preparation is formulated for systemic administration, preferably for intravenous, intramuscular, subcutaneous, intradermal, transdermal, or oral administration.

9. The pharmaceutical preparation for use according to any one of claims 1 to 8, wherein said pharmaceutical preparation is administered to the subject as a spray, a powder, a gel, an ointment, a cream, a foam, or a liquid solution, a lotion, a gargle solution, an aerosolized powder, an aerosolized liquid formulation, granules, capsules, drops, tablet, syrup, lozenge, or a preparation for infusion or injection.

10. The pharmaceutical preparation for use according to any one of claims 1 to 9, wherein said pharmaceutical preparation is applied in an effective amount into a subject suffering from FSGS, or being at risk of developing FSGS.

11. The pharmaceutical preparation for use according to any one of claims 1 to 10, wherein the preparation is administered as the sole substance, or wherein the preparation is combined with a further preparation comprising one or more active substances.

12. The pharmaceutical preparation for use according to any one of claims 1 to 11, wherein the preparation is administered in combination with an active agent selected from the group consisting of antiviral drugs, anticoagulants, immune modulators, antibody preparations from human sources, monoclonal antibodies, intensive care medications, antihypertensive agents, statins, vasodilators, steroids, cytotoxic drugs, diuretics, non-steroidal anti-inflammatory drugs (NSAIDs), cholesterol or triglyceride reducing agents, and PPAR agonists.

## Patentansprüche

1. Pharmazeutische Zubereitung umfassend Clopidogrel oder ein Salz oder Solvat davon zur Verwendung bei der Behandlung eines nicht-diabetischen Subjekts, das unter fokal-segmentaler Glomerulosklerose (FSGS) leidet.

2. Pharmazeutische Zubereitung zur Verwendung nach Anspruch 1, wobei Clopidogrel als der einzige aktive Wirkstoff vorliegt.

3. Pharmazeutische Zubereitung zur Verwendung nach Anspruch 1 oder 2, wobei die FSGS eine primäre FSGS ist.

4. Pharmazeutische Zubereitung zur Verwendung nach einem der Ansprüche 1 bis 3, wobei die pharmazeutische Zubereitung ein medizinisches Produkt oder ein Arzneimittelprodukt ist, das Clopidogrel und einen pharmazeutisch annehmbaren Träger umfasst.

5. Pharmazeutische Zubereitung zur Verwendung nach einem der Ansprüche 1 bis 4, wobei die Zubereitung systemisch verabreicht wird.

6. Pharmazeutische Zubereitung zur Verwendung nach einem der Ansprüche 1 bis 5, wobei Clopidogrel die Struktur 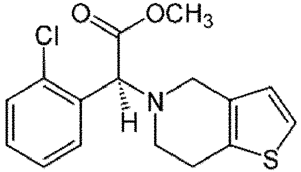 umfasst.

7. Pharmazeutische Zubereitung zur Verwendung nach Anspruch 6, wobei die Zubereitung etwa 10 bis 400 mg Clopidogrel umfasst.

8. Pharmazeutische Zubereitung zur Verwendung nach einem der Ansprüche 1 bis 7, wobei die pharmazeutische Zubereitung für die systemische Verabreichung formuliert ist, vorzugsweise für die intravenöse, intramuskuläre, subkutane, intradermale, transdermale oder orale Verabreichung.

9. Pharmazeutische Zubereitung zur Verwendung nach einem der Ansprüche 1 bis 8, wobei die pharmazeutische Zubereitung an das Subjekt als ein Spray, ein Pulver, ein Gel, eine Salbe, eine Creme, ein Schaum oder eine flüssige Lösung, eine Lotion, eine Gurgellösung, ein zerstäubtes Pulver, eine zerstäubte flüssige Formulierung, als Granulat, Kapseln, Drops, Tablette, Sirup, Lutschtablette oder eine Zubereitung zur Infusion oder Injektion verabreicht wird.

10. Pharmazeutische Zubereitung zur Verwendung nach einem der Ansprüche 1 bis 9, wobei die pharmazeutische Zubereitung in einer wirksamen Menge in einem Subjekt angewendet wird, das unter einer FSGS leidet, oder für welches das Risiko besteht, eine FSGS zu entwickeln.

11. Pharmazeutische Zubereitung zur Verwendung nach einem der Ansprüche 1 bis 10, wobei die Zubereitung als einzige Substanz verabreicht wird, oder wobei die Zubereitung mit einer weiteren Zubereitung kombiniert wird, die eine oder mehrere aktive Substanzen umfasst.

12. Pharmazeutische Zubereitung zur Verwendung nach einem der Ansprüche 1 bis 11, wobei die Zubereitung in Kombination mit einem aktiven Wirkstoff verabreicht wird, der aus der Gruppe ausgewählt ist, die aus antiviralen Arzneimitteln, Antikoagulanzien, Immun-Modulatoren, Antikörperzubereitungen aus menschlichen Quellen, monoklonalen Antikörpern, intensivmedizinischen Medikationen, antihypertensiven Mitteln, Statinen, Vasodilatoren, Steroiden, zytotoxischen Arzneimitteln, Diuretika, nichtsteroidalen anti-inflammatorischen Arzneimitteln (NSAIDs), cholesterin- oder triglyceridsenkenden Mitteln, und PPAR-Agonisten besteht.

## Revendications

1. Préparation pharmaceutique comprenant du clopidogrel, ou un sel ou solvate de celui-ci, destinée à être utilisée dans le traitement d'un sujet non diabétique souffrant de glomérulosclérose segmentaire focale (FSGS).

2. Préparation pharmaceutique destinée à être utilisée selon la revendication 1, dans laquelle le clopidogrel est présent en tant qu'unique principe actif.

3. Préparation pharmaceutique destinée à être utilisée selon la revendication 1 ou 2, dans laquelle la FSGS est une FSGS primaire.

4. Préparation pharmaceutique destinée à être utilisée selon l'une quelconque des revendications 1 à 3, la préparation pharmaceutique étant un produit médicinal ou un produit médicamenteux, comprenant du clopidogrel et un vecteur pharmaceutiquement acceptable.

5. Préparation pharmaceutique destinée à être utilisée selon l'une quelconque des revendications 1 à 4, la préparation étant administrée de manière systémique.

6. Préparation pharmaceutique destinée à être utilisée selon l'une quelconque des revendications 1 à 5, dans laquelle le clopidogrel comprend la structure

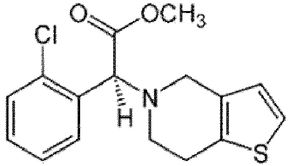

7. Préparation pharmaceutique destinée à être utilisée selon la revendication 6, la préparation comprenant environ 10 à 400 mg de clopidogrel.

8. Préparation pharmaceutique destinée à être utilisée selon l'une quelconque des revendications 1 à 7, ladite préparation pharmaceutique étant formulée pour une administration systémique, de préférence pour une administration intraveineuse, intramusculaire, sous-cutanée, intradermique, transdermique ou orale.

9. Préparation pharmaceutique destinée à être utilisée selon l'une quelconque des revendications 1 à 8, ladite préparation pharmaceutique étant administrée au sujet sous la forme d'un spray, d'une poudre, d'un gel, d'une pommade, d'une crème, d'une mousse, ou d'une solution liquide, d'une lotion, d'une solution de gargarisme, d'une poudre aérosolisée, d'une formulation liquide aérosolisée, de granulés, de capsules, de gouttes, d'un comprimé, d'un sirop, d'une pastille, ou d'une préparation pour perfusion ou injection.

10. Préparation pharmaceutique destinée à être utilisée selon l'une quelconque des revendications 1 à 9, ladite préparation pharmaceutique étant appliquée en une quantité efficace dans un sujet souffrant de FSGS, ou présentant un risque de développer une FSGS.

11. Préparation pharmaceutique destinée à être utilisée selon l'une quelconque des revendications 1 à 10, la préparation étant administrée sous la forme d'une seule substance, ou la préparation étant combinée à une autre préparation comprenant un ou plusieurs principes actifs.

12. Préparation pharmaceutique destinée à être utilisée selon l'une quelconque des revendications 1 à 11, la préparation étant administrée en combinaison avec un principe actif choisi dans le groupe constitué par les médicaments antiviraux, les anticoagulants, les modulateurs immunitaires, les préparations d'anticorps provenant de sources humaines, les anticorps monoclonaux, les médicaments de soins intensifs, les agents antihypertenseurs, les statines, les vasodilatateurs, les stéroïdes, les médicaments cytotoxiques, les diurétiques, les médicaments anti-inflammatoires non stéroïdiens (NSAID), les agents réducteurs de cholestérol ou de triglycéride, et les agonistes de PPAR.
